# EUROPEAN PATENT APPLICATION

(11) **EP 4 023 290 A1**
(43) Date of publication of application: **06.07.2022**
(21) Application number: 20858765.9
(22) Date of filing: 21.08.2020
(51) Int. Cl.: A61P 1/00, A61P 1/04, A61P 3/10, A61P 37/08, A61P 17/00, A61K 9/10, A61K 47/04, A61K 47/26, A61K 47/32, A61K 47/36, A61K 35/74, A61K 35/741, A61K 35/744

(54) **BIOLOGICAL INTRODUCTION AID AND METHOD OF USE THEREOF**

(30) Priority: 26.08.2019 JP 2019154049; 27.03.2020 JP 2020058704
(71) Applicant: Shinbiosis Corporation, Osaka City, Osaka, 534-0025 (JP)
(72) Inventor: SHIMIZU Shin, Osaka City Osaka 559-0013 (JP)
(74) Representative: TBK
(86) International application number: PCT/JP2020/031671
(87) International publication number: WO 2021/039645

(57) **Abstract**

The aim of the present invention is to provid an assistant agent for assisting introduction into a living body to be used to introduce an active substance into a living body, and a composition that includes the same. The present invention is directed to an assistant agent for assisting introduction into a living body or an assistant agent for a drug delivery system that is to be used to introduce (I)-(i) together with (I)-(ii) into a living body and that includes (II) and (III) below, or a composition containing an active substance that includes (I)-(i) to (III):
(I)-(i) an active substance;
(I)-(ii) an active substance protective agent;
(II) a solvent; and
(III) nano-sized or smaller (smaller than 1 micron) gas bubbles.

## Description

### Technical Field

The present invention relates to an "assistant agent for assisting introduction into a living body" that can be used as an assistant agent for a drug delivery system capable of more reliably delivering a "complex containing an active substance (e.g., what is called a pharmaceutical formulation)" that includes an "active substance" and an "active substance protective agent" such as an excipient (a vehicle) to the inside of a living body.

Also, the present invention relates to a "composition containing an active substance" that can be more reliably introduced to the inside of a living body.

Furthermore, the present invention relates to a "composition for preventing and/or treating a disease or improving a physical constitution and/or physical condition" that can more reliably exhibit a function, efficacy, or effect in a living body.

### Background Art

A concept of what is called drug delivery has become known as an attempt to cause an active substance such as a drug to more effectively exhibit the function, efficacy, or effect thereof in a living body.

In particular, intestinal drugs containing intestinal bacteria and the like and other microbial formulations do not exhibit their effects as expected, and therefore, various drug delivery systems have been under research and development. However, satisfactory drug delivery systems have not been realized up to now.

On the other hand, apparatuses for generating minute gas bubbles having a diameter of 100 µm or less, that is, what is called microbubbles, nanobubbles having a diameter smaller than 1 µm, or the like have been developed (Patent Document 1, etc.), and solutions containing such gas bubbles have come into use in various fields such as the medical field, the agricultural field, the fishery field, the aquacultural field, and the like.

However, there has been substantially no technical idea of using a solution containing minute gas bubbles (what is called nanobubble water) to introduce bacteria in a live state into a living body, and it goes without saying that it has not been proved that such a solution actually improves the efficiency of introduction into a living body.

### Citation List

### Patent Document

Patent Document 1: JP 2012-582A

### Summary of Invention

### Technical Problem

The inventor of the present invention developed a method of using a solution containing minute gas bubbles (what is called nanobubble water) to cause bacteria to engraft in a living body in a live state (PCT/2019/7574). In addition, the inventor found that nanobubble water exhibited what is called a drug delivery effect on even intestinal bacteria protected by an excipient, and that nanobubbles could sufficiently exhibit a protective effect on even a "protein" or "low molecular weight organic compound", which is so small that it is difficult to cover it by nanobubbles, by protecting the protein or low molecular weight organic compound with a kind of "protective agent" such as an excipient (a vehicle), and the present invention was thus accomplished. It is an object of the present invention to provide an "assistant agent for assisting introduction into a living body" that can be used as an assistant agent for a drug delivery system capable of more reliably delivering an active substance together with an active substance protective agent such as an excipient, a metal particle, or the like to the inside of a living body, a "composition containing an active substance" that contains such an assistant agent, a "composition for preventing and/or treating a disease or improving a physical constitution and/or physical condition" that contains such a composition, a "method for manufacturing such a composition", and the like.

### Solution to Problem

The above-described object is achieved by the following first to fourteenth aspects of the present invention.

### First Aspect

An assistant agent for assisting introduction into a living body, the assistant agent to be used to introduce (I)-(i) together with (I)-(ii) into a living body, and including (II) and (III) below.

(I)-(i) an active substance
(I)-(ii) an active substance protective agent
(II) a solvent; and
(III) nano-sized or smaller (smaller than 1 micron (1 µm)) gas bubbles

### Second Aspect

The assistant agent for assisting introduction into a living body according to the first aspect, wherein a gas component in the gas bubbles comprises one or two or more kinds of gasses listed below.

(i) air
(ii) hydrogen
(iii) nitrogen
(iv) ozone
(v) oxygen
(vi) carbon dioxide; and
(vii) argon

### Third Aspect

The assistant agent for assisting introduction into a living body according to the first or second aspect, wherein (I)-(ii) active substance protective agent is at least one kind of protective agent listed below.

### (I)-(ii)-(a) an excipient (vehicle)

### Fourth Aspect

The assistant agent for assisting introduction into a living body according to any one of the first to third aspects, wherein (I)-(i) active substance includes at least one or more kinds of microorganisms.

### Fifth Aspect

The assistant agent for assisting introduction into a living body according to the fourth aspect, wherein at least one kind of the microorganism is a microorganism derived from a living body.

### Sixth Aspect

The assistant agent for assisting introduction into a living body according to the fifth aspect, wherein at least one kind of the microorganism derived from a living body is an intestinal bacterium.

### Seventh Aspect

The assistant agent for assisting introduction into a living body according to any one of the first to third aspects, wherein the active substance of (I)-(i) includes at least one or more kinds of organic compounds.

### Eighth Aspect

The assistant agent for assisting introduction into a living body according to any one of the first to seventh aspects, further including (I)-(ii) below:
(I)-(ii) an active substance protective agent.

### Ninth Aspect

An assistant agent for a drug delivery system, including (II) and (III) below:
(II) a solvent; and
(III) nano-sized or smaller (smaller than 1 micron) gas bubbles.

### Tenth Aspect

A composition containing an active substance, including (I)-(i) to (III) below.

(I)-(i) an active substance
(I)-(ii) an active substance protective agent
(II) a solvent; and
(III) nano-sized or smaller (smaller than 1 micron) gas bubbles

### Eleventh Aspect

A composition for preventing and/or treating a disease or improving a physical constitution and/or physical condition, including the composition according to the tenth aspect.

### Twelfth Aspect

A method for manufacturing the composition according to the tenth or eleventh aspect, including at least steps of (1) to (3) below.

(1) a step of preparing (I)-(i) protected by (I)-(ii)
(2) a step of generating gas bubbles of (III) in a solvent of (II) ; and
(3) a step of dispersing and/or dissolving (I)-(i) in (II)

(I)-(i) an active substance
(I)-(ii) an active substance protective agent
(II) a solvent; and
(III) nano-sized or smaller (smaller than 1 micron) gas bubbles

### Thirteenth Aspect

A method for preventing and/or treating a disease or improving a physical constitution and/or physical condition by introducing the composition according to the eleventh aspect into a living body.

### Fourteenth Aspect

A method for causing an active substance to exhibit a function, efficacy, or effect thereof in a living body by using the assistant agent for assisting introduction into a living body according to any one of the first to ninth aspects to introduce (I)-(i) below together with (I)-(ii) below.

(I)-(i) an active substance; and
(I)-(ii) an active substance protective agent

### Advantageous Effects of Invention

The assistant agent for assisting introduction into a living body of the present invention can be used as an assistant agent for a drug delivery system (DDS) capable of more reliably delivering an active substance together with an active substance protective agent such as an excipient, or the like to the inside of a living body.

The composition containing an active substance of the present invention can be more reliably introduced to the inside of a living body.

The composition for preventing and/or treating a disease or improving a physical constitution and/or physical condition of the present invention can more reliably exhibit a function, efficacy, or effect in a living body, and is thus useful in preventing and/or treating a disease or improving a physical constitution and/or physical condition.

### Brief Description of Drawings

FIG. 1 is a diagram showing the results of measurements of the sizes, numbers, and the like of gas bubbles in an "assistant agent A for assisting introduction into a living body" of Example A.
FIG. 2 is a diagram showing changes in the ratio of Bacteroides in the microbiota (intestinal flora) in feces of mice to which "composition containing an active substance" of Example 1 or Comparative Example 1 was administered.
FIG. 3 is a diagram showing changes in the ratio of Clostridium cluster XIVab in the microbiota (intestinal flora) in feces of mice to which "composition containing an active substance" of Example 1 or Comparative Example 1 was administered.
FIG. 4-1 is a diagram showing the results of confirming, through fluorescence intensity, a glucose absorption suppressing effect exhibited when "complexes containing an active substance (intestinal bacteria + excipient)" were fed to intestinal epithelial cells with "assistant agents for assisting introduction into a living body" of Example B or Comparative Example C.
FIG. 4-2 is a diagram showing the ratio (glucose uptake ratio (%)) of the "relative fluorescence intensity of Example B (nanobubble water B)" to the "relative fluorescence intensity of Comparative Example C (physiological saline solution)" at each time point shown in FIG. 4-1.
FIG. 5 is a diagram showing the experimental protocol in accordance with which a "complex containing an active substance (intestinal bacteria + excipient)" is fed to induced diabetes model mice (STZ-mice) with the "assistant agents for assisting introduction into a living body" of Example B and Comparative Example C.
FIG. 6-1 is a diagram showing the changes in blood glucose level when "complex containing an active substance (intestinal bacteria + excipient)" was fed to induced diabetes model mice (STZ-mice) with the "assistant agent for assisting introduction into a living body" of Example B or Comparative Example C.
FIG. 6-2 is a diagram showing the plasma insulin levels when "complex containing an active substance (intestinal bacteria + excipient)" was fed to induced diabetes model mice (STZ-mice) with the "assistant agent for assisting introduction into a living body" of Example B or Comparative Example C.

### Description of Embodiments

Hereinafter, the present invention will be described in detail.

### Assistant Agent for Assisting Introduction into Living Body of the Present Invention

An "assistant agent for assisting introduction into a living body" of the present invention is used to introduce an active substance of (I)-(i) together with an active substance protective agent of (I)-(ii) into a living body, and is characterized by including (II) and (III) below.

In the present invention, this agent may also be referred to as "nanobubble water (NB water, NB, or the like)".

(I)-(i) an active substance
(I)-(ii) an active substance protective agent
(II) a solvent
(III) nano-sized or smaller (smaller than 1 µm) gas bubbles

It should be noted that (I)-(i) and (I)-(ii) will be described in the section "Composition Containing Active Substance" of the present invention below.

### Solvent of (II) Used in the Present Invention

There is no particular limitation on the solvent of (II) used in the present invention, and purified water, a physiological saline solution, mineral water, soft drink, or the like may be used.

A physiological saline solution or the like may be used as (II) from the viewpoint of improving familiarity of the "active substance" of (I)-(i) to a living body and engraftment thereof in a living body and in view of an antiseptic effect and the like.

On the other hand, when an apparatus for generating gas bubbles of (III) in the solvent of (II) is made of stainless steel or the like, it may be preferable to use purified water, mineral water, or the like from the viewpoint of preventing the apparatus from getting rusty.

### Nano-Sized or Smaller Gas Bubbles of (III) Used in the Present Invention

Preferable examples of a "gas component" in the nano-sized or smaller gas bubbles of (III) used in the present invention include gasses as listed below, but are not necessarily limited thereto.

### Kinds of Gasses in Gas Bubbles

It is preferable that the "gas component" in the gas bubbles comprises one or two or more kinds of gasses listed below.

(III)-(i): air
(III)-(ii): hydrogen
(III)-(iii): nitrogen
(III)-(iv): ozone
(III)-(v): oxygen
(III)-(vi): carbon dioxide
(III)-(vii): argon

Using air of (III)-(i) alone is practical and preferable because there is no need to prepare special "gas components" such as those of (III)-(ii) to (III)-(vii), for example.

It should be noted that, when "air" or "a mixed gas of two or more kinds of gasses" is introduced into the solvent of (II), it is not easy to accurately measure the "ratio between the gas components enclosed" in the "gas bubbles (III) generated in the solvent (II)" with current technologies.

The reason for this is that the "amount" of the gas component that can dissolve in the solvent (II) and the "speed" at which the gas component can dissolve in the solvent (II) depend on the "gas components" in the process in which the gas bubbles (III) are generated in the solvent (II), and it is not easy to accurately determine the "kinds" of and "ratio" between the "gas components" enclosed in the generated gas bubbles (III).

However, when a "gas component (referred to as "X" for the sake of convenience)" is used alone or in combination with air, for example, it is thought that the "ratio of the gas component (X) enclosed" in the "gas bubbles (III) generated in the solvent (II)" should increase compared with a case where only air is used, and thus the characteristics of the "gas component (X)" as described above can be further utilized.

It should be noted that it is preferable to increase the ratio of hydrogen in the gas bubbles compared with a case where air is used alone because the following advantages are expected.
1. The oxidation-reduction potential (target: -150 mV±15 mV) of nanobubble water of the present invention in which hydrogen is used is as low as the oxidation-reduction potential (-50 mV to -250 mV) of the intestinal tract, and thus it is thought that the "active substance" of (I)-(i) covered with the nanobubble water is easy to engraft in the intestines.
2. When the intestinal tract is inflamed, the oxidation-reduction potential tends to increase, and thus the introduced (I) is apt to be repelled by the immunity of a transplantation target due to an increased immune response. However, it is thought that this response can be suppressed by an anti-inflammatory action due to the low oxidation-reduction potential of hydrogen.
3. For example, when "intestinal bacteria" are used as the "active substance", both facultative anaerobes and obligatory anaerobes, which are main groups of "intestinal bacteria", can be maintained in a state in which they are made inactive but are not killed by using the "nanobubble water" of the present invention that contains hydrogen molecules, and thus it is thought that a bacterial solution can be stored in a state in which the bacteria lie dormant for a relatively long period of time without disturbing the balance between the different bacteria in the bacterial solution, until the bacteria become active again inside the intestinal tract, which is an appropriate environment, after transplantation.

In order to set the ratio of hydrogen in the gas bubbles higher than that of air, a method of using hydrogen alone as well as a method of using air and hydrogen together, and the like can be used.

In this case, air and a gas including only hydrogen may be enclosed simultaneously, but a method in which the concentration of hydrogen enclosed together with air is gradually increased, a method in which only air is used at first and then hydrogen is enclosed in a latter stage, or the like may also be used. It is thought that using these methods makes it possible to minimize the loss of hydrogen due to dissolution in the solvent and enclose a larger amount of hydrogen in gas bubbles.

Although there is no particular limitation on the ratio between air and hydrogen when they are used simultaneously, for example, it is considered preferable to set the amount of "(ii) hydrogen" used in an enclosing operation to be ten or more times the amount of "(i) air" used therein, and more preferable to set the amount of "(ii) hydrogen" to be one hundred or more times the amount of "(i) air" used therein.

### Size of Gas Bubbles

The "(III) nano-sized or smaller (smaller than 1 micron) gas bubbles" used in the "assistant agent for assisting introduction into a living body" of the present invention need to be mainly nano-sized or smaller (smaller than 1 micron) gas bubbles. On the other hand, the specific size applicable to every case cannot be determined in general since it can be flexibly changed, as circumstances demand, in accordance with the kind, size, or the like of (1)-(i) that is protected by (I)-(ii) and is to be introduced into a living body with this assistant agent. However, in order to more reliably protect the periphery of "(1)-(i) protected by (I)-(ii)" through a covering technique or the like, it is more preferable that the size is small enough to entirely protect "(1)-(i) protected by (I)-(ii)".

Conversely, even in a case where the "(1)-(i) active substance", which is to be introduced into a living body with assistance, is smaller in size than gas bubbles and is thus difficult to protect, when the "(1)-(i) active substance" is protected by the "(I)-(ii) an active substance protective agent" and is thus increased in size, the assistant agent for assisting introduction into a living body of the present invention can be used.

Specifically, in order to enhance the effect of introduction into a living body compared with a conventional case, the size of the "(III) nano-sized or smaller (smaller than 1 micron) gas bubbles" is preferably, for example, about 900 nm or less, and more preferably several hundred nm or less. The inventor of the present invention confirmed that, when the size is particularly several ten nm or less, further, several nm or less, the speed at which (I) is introduced into a living body and the ratio of (I) introduced into a living body are significantly increased.

However, the effect of the present invention can be sufficiently exhibited even when the size of gas bubbles is about several hundred nm, and therefore, it is sufficient in practice that the size of gas bubbles is determined in view of the balance with production cost of reducing the size of gas bubbles.

### Ratio of Nano-sized or smaller Gas Bubbles to All Gas Bubbles

"Nano-sized or smaller gas bubbles (III)" need not necessarily account for 100% of the gas bubbles in the "assistant agent for assisting introduction into a living body".

However, it is thought that the reason why the "(I)-(i) active substance" can more reliably exhibit the function, efficacy, or effect in a living body by using the "assistant agent for assisting introduction into a living body of the present invention" is that "nano-sized or smaller gas bubbles (III)" that are smaller in size than "(I)-(i) protected by (I)-(ii)" protect the surface or periphery of "(I)-(i) protected by (I)-(ii)" by, for example, covering it, or more directly protect the active substance by entering the inside through gaps and the like present in the surface or periphery of "(I)-(i) protected by (I)-(ii)", and thus assist the introduction of the active substance into a living body. Therefore, it is preferable that a large number of "nano-sized or smaller gas bubbles (III)" are contained such that they can protect at least the entire surface or periphery of each "(I)-(i) protected by (I)-(ii)".

Furthermore, it is desirable that the ratio of the "nano-sized or smaller gas bubbles (III)" is as high as possible because the denaturation of the composition caused by the collapse of supranano-sized gas bubbles is suppressed, and the ratio of the "active substance of (I)-(i)" introduced into a living body is further increased.

In general, the size of the gas bubbles is not always uniform, and a distribution to some extent of gas bubble diameters is commonly observed.

Accordingly, it is thought that the following can be used as a specific guide: if the "average diameter" of the gas bubbles is smaller than the entire diameter of the "(I)-(i) protected by (I)-(ii)" to be used, there will also be a large number of gas bubbles that are small enough to protect "(I)-(i) protected by (I)-(ii)". For example, it is preferable to use a solution containing gas bubbles with an average air bubble diameter (distance across) of smaller than 1000 nm, and more preferably an average air bubble diameter of 900 nm or less.

### Number of Gas Bubbles in Assistant Agent for Assisting Introduction into Living Body

The larger number of gas bubbles the "assistant agent for assisting introduction into a living body" of the present invention contains, the more desirable it is.

Although not necessarily applicable to every case since the specific number of gas bubbles depends on the kind or concentration of "(I)-(i) protected by (I)-(ii)", it was confirmed that several thousand to several hundred million gas bubbles per milliliter, which can be commonly generated by using a known generation apparatus capable of generating the "nano-sized or smaller gas bubbles (III)", are sufficient.

However, it is thought that several ten million to several hundred million gas bubbles per milliliter are more preferable.

It should be noted that an example of a method of measuring minute gas bubbles is a method known as an electrical sensing zone method based on the Coulter principle, which will be described below. Specific examples thereof include methods in which "Multisizer 3", "Multisizer 4", "Multisizer 4e", (these are manufactured by Beckman Coulter, Inc.) or the like are used.

With a method based on the Coulter principle, the distributions of the diameters and numbers of gas bubbles are measured as follows: in a state in which a certain amount of an electrolyte solution flows inside a cylinder (manometer) provided with one or more minute holes (apertures), a D.C. voltage is applied using electrodes installed on the inside and the outside of the manometer (the electrode on the inside is a negative electrode, and the electrode on the outside is a positive electrode), and a change in electrical resistance between the two electrodes that is generated when particles (e.g., gas bubbles) pass through a sensing zone (aperture sensing area) is measured.

### Method for Manufacturing Assistant Agent for Assisting Introduction into Living Body

The "assistant agent for assisting introduction into a living body" of the present invention can be manufactured by generating gas bubbles of (III) in a solvent of (II).

Examples of a method for "generating gas bubbles of (III) in a solvent of (II)" include, but are not limited to, the following techniques and methods in which these techniques are used in combination.

### Gas-liquid mixture shearing technique:

This is a technique in which gas is rotated at a high speed together with a liquid.

### Ultrasonic technique:

This is a technique in which a shock wave is applied to or cavitation is allowed to occur in a liquid to further collapse gas bubbles that have been generated.

### Pressurized dissolution technique:

This is a technique in which gas bubbles are generated by pressurizing gas and a liquid and discharging them in one burst.

### Micropore technique:

This is a technique in which gas is supplied while pressure is applied thereto using an orifice or the like.

### Electrolytic technique:

This is a technique in which gas is generated from a thin wire immersed in an aqueous solution.

Among the above-mentioned techniques, the "gas-liquid mixture shearing technique" is preferable because stable nano-sized or smaller gas bubbles can be generated by further performing shearing processing on microbubbles.

Specifically nanobubbles can be generated by using commercially available apparatuses of 1) and 2) below together, for example.
1) Generation of gas bubbles with a diameter of 1 micrometer or less using a micro-nanobubble generation apparatus "BUVITAS (registered trademark) HYK-25" manufactured by Kyowa Kisetsu (rotation shearing technique)
2) Formation of nanobubbles from micro-nanobubbles using "vG7 (registered trademark)" manufactured by AYAWA Co., Ltd. (stainless-steel filter)

It should be noted that using an "ultrafine bubble generating apparatus" that was developed by the inventor of the present invention and that is disclosed in the patent application below is particularly preferable because nanobubble water containing gas bubbles with a diameter of smaller than 1 µm (e.g., gas bubbles with an average diameter in the order of several nm to several hundred nm) at a high concentration can be efficiently produced.

### Japanese Patent Application 2020-57176

The above-mentioned "assistant agent for assisting introduction into a living body" may contain the active substance protective agent of (I)-(ii) as one of the constituent components thereof in advance and, for example, metal particles of (I)-(ii)-(b) may be used to form metal colloidal particles in the solvent of (II).

The metal colloidal particles can be formed by using a method of reducing a metal-containing compound such as a metal salt or the like as well as by dispersing metal particles in a solvent.

It is sufficient that metal particles are contained in such an amount that is enough to protect the active substance. That is, the content of the metal particles depends on the amount of the active substance to be introduced, and is thus not particularly limited.

### Application of Assistant Agent for Assisting Introduction into Living Body

The "assistant agent for assisting introduction into a living body" of the present invention is used to introduce the "(I)-(i) active substance" together with the "(I)-(ii) active substance protective agent" into a living body.

The following are specific examples of the application of the "assistant agent for assisting introduction into a living body":
A) the "assistant agent for assisting introduction into a living body" is used to manufacture the "composition containing an active substance" of the present invention by dispersing and/or dissolving "(I)-(i)" together with "(I)-(ii)" into the assistant agent itself;
B) "(I)-(i)" is dispersed and/or dissolved in the "assistant agent for assisting introduction into a living body" together with "(I)-(ii)" just before being introduced into a living body; and
C) the "assistant agent for assisting introduction into a living body" is administered to a living body independently of "(I)-(i)" and "(I)-(ii)" simultaneously with or at a time point slightly different from the administration of "(I)-(i)" and "(I) - (ii)" .

In order to suppress the deactivation of the "(I)-(i) active substance" as far as possible or to minimize, for example, the loss of nano-sized or smaller gas bubbles due to collapse thereof in the "assistant agent for assisting introduction into a living body", the application described in B) or C) above is preferable.

The reason why the "assistant agent for assisting introduction into a living body" can be independently administered is that gas bubbles have properties such that, when a certain solid substance is present together with gas bubbles in a solvent, the gas bubbles spontaneously attach to the periphery of the solid substance.

That is, it is thought that, even when the "assistant agent for assisting introduction into a living body" is administered independently of "(I)-(i)" and "(I)-(ii)" simultaneously with or at a time point slightly different from the administration of "(I)-(i)" and "(I)-(ii)", "(I)-(i)" and "(I)-(ii)" are immediately covered by "nano-sized or smaller gas bubbles" contained in the "assistant agent for assisting introduction into a living body" as long as "(I)-(i)" and "(I)-(ii)" come into contact with the "assistant agent for assisting introduction into a living body" before moving deeper into a living body.

The "assistant agent for assisting introduction into a living body" of the present invention can also be used as an "assistant agent for a drug delivery system" of the present invention, which will be described later.

### Method of Using Assistant Agent for Assisting Introduction into Living Body

It is thought that, when the "assistant agent for assisting introduction into a living body" and "(I)-(i) protected by (I)-(ii)" are slightly stirred or mixed, "nano-sized or smaller gas bubbles (III)" spontaneously accumulate or the like so as to loosely surround or cover the periphery of "(I)-(i) protected by (I)-(ii)" and thus protect it, and, depending on the situation, the "nano-sized or smaller gas bubbles (III)" further enter the inside of "(I)-(i) protected by (I)-(ii)" through gaps and the like present in the surface or periphery thereof and thus more directly and reliably protect the active substance of (I)-(i).

It is sufficient that "(I)-(i) protected by (I)-(ii)" is dispersed and/or dissolved at a concentration such that "(I)-(i) protected by (I)-(ii)" is sufficiently covered by nano-sized gas bubbles in the (II) solvent.

The reason for this is that, when microorganisms derived from a living body such as intestinal bacteria or the like are used as the active substance, for example, it is thought that the more sufficiently "(I)-(i) protected by (I)-(ii)" is covered by nano-sized gas bubbles, the more rapidly and reliably the microorganisms can engraft in a living body without being inhibited by a mucous membrane or the like of the intestinal wall made of mucopolysaccharides and the like.

Although the specific concentration applicable to every case cannot be determined in general since it depends on the kind and size of "(I)-(i) protected by (I)-(ii)" or the number of gas bubbles in the "assistant agent for assisting introduction into a living body", when a powdered drug constituted by powder that contains several kinds of microorganisms derived from a living body such as intestinal bacteria or the like is used as the active substance, for example, the amount of "(I)-(i) protected by (I)-(ii)" per 1 ml of the "assistant agent for assisting introduction into a living body" is preferably, for example, about 0.5 to 20 mg, and more preferably 1 to 10 mg.

It should be noted that a "living body" refers to "live bodies" of humans or non-human animals in general, but also encompasses portions of a living body (e.g., "organs", "nails", "head hair and other body hair", "blood", "body fluids such as lymph fluid", "cells", and the like) that are prepared based on the premise of being returned to the living body or that are to be used for a test or investigation (i.e., prepared based on the premise of not being returned to the living body) in addition to such a "live body" itself.

The "cells" encompass those obtained by culturing or cloning extracted cells, and those obtained through differentiation induction of iPS cells, ES cells, other multipotent stem cells, and the like.

The "organs" encompass not only those extracted from "live bodies", but also those obtained through proliferation or differentiation induction of a portion of an organ or cells extracted from an organ, and those obtained through differentiation induction of iPS cells, ES cells, other multipotent stem cells, and the like.

Moreover, "introduction into a living body" means that the introduced active substance is brought into a state of being capable of exhibiting its function, efficacy, or effect in a living body by being physically bound to the living body through engraftment, attachment, adsorption, or the like, as well as, for example, by being allowed to be present inside or outside the blood vessels, in cavities in the living body (in the intestinal tract), and the like, or by being added to a cell culture solution, for example. Substantially all known introduction techniques including direct injection techniques for blood vessels, subcutaneous tissues, and intracutaneous tissues can be employed.

Specific examples of the administration route include routes via the oral cavity, the eyes, the ears, the nose, the vagina, the urethra, the skin, and the anus. Of these, the introduction via the mucous membrane is preferable, and the introduction via the intestinal wall is particularly preferable.

It should be noted that the "assistant agent for assisting introduction into a living body" of the present invention may also contain various additives and the like in addition to (II) and (III) as long as the object of the present invention is not hindered.

### Assistant Agent for Drug Delivery System of the Present Invention

An "assistant agent for a drug delivery system (also referred to as a "DDS assistant agent" hereinafter)" of the present invention is characterized by including (II) and (III) below:
(II) a solvent; and
(III) nano-sized or smaller (smaller than 1 micron) gas bubbles.

Using this assistant agent makes it possible to further improve the delivery effects of known and unknown drug delivery systems (DDSs).

### Composition Containing Active Substance of the Present Invention

The "composition containing an active substance" of the present invention is characterized by including (I)-(i) to (III) below:
(I)-(i) an active substance;
(I)-(ii) an active substance protective agent;
(II) a solvent; and
(III) nano-sized or smaller (smaller than 1 micron) gas bubbles.

(I)-(i) and (I)-(ii) will be described below in the stated order.

### (I)-(i) Active Substance

The "active substance" encompasses all substances that have activity useful for a living body, and examples thereof include various organic compounds ranging from low molecular weight organic molecules to high molecular weight organic molecules, microorganisms, and inorganic substances such as minerals, and the like.

The organic compounds encompass natural substances, in vivo substances, semi-artificial substances obtained by modifying these substances, artificial substances, and the like.

Specific examples thereof include various peptides, polypeptides, proteins, amines, steroids, various hormones (endocrine therapeutic agents), cytokines, neurotransmitters, autacoids, derivatives thereof, modifications thereof, and the like, and other various low molecular weight organic compounds and the like (which may be natural substances, semi-artificial substances, or artificial substances).

The hormones refer to physiologically active substances that are secreted in a body in response to information from inside and outside of a living body, and examples thereof include protein (peptide) hormones, amino-acid derivative hormones, steroid hormones, and the like.

### Protein (peptide) hormones:

Specific examples of the protein (peptide) hormones include inhibin, parathyroid hormones, calcitonin, a thyrotropin-releasing hormone (TRH), a thyroid-stimulating hormone (TSH), vasopressin (antidiuretic hormone: ADH), a corticotrophin-releasing hormone (CRH), an adrenocorticotropic hormone (ACTH), a gonadotropin-releasing hormone (GnRH), gonadotropic hormones (a luteinizing hormone (LH), a follicle-stimulating hormone (FSH), chorionic gonadotropin), prolactin (PRL), melatonin, oxytonin, insulin, glucagon, somatostatin, pancreatic polypeptides, growth hormone-releasing hormones, growth hormones, and the like.

### Amino-acid derivative hormones:

Specific examples of the amino-acid derivative hormones include thyroid hormones such as thyroxine, and adrenomedullary hormones (catecholamines) such as adrenaline, noradrenaline, dopamine, and the like.

### Steroid hormones:

Specific examples of the steroid hormones include male (testis) hormones including androgen such as testosterone and the like, female (ovarian) hormones including estrogen (follicle hormone), progesterone (corpus luteum hormone), and the like, adrenocortical hormones such as mineral corticoid (aldosterone), glucocorticoid (cortisol), and the like, and activated vitamin D.

It should be noted that a hormone drug as used in the present invention encompasses the above-mentioned natural hormones, derivatives thereof, artificial mutants thereof, and the like.

The term "microorganisms" as used in the present invention means all minute organisms that exist in nature.

Typical examples thereof include what is called "microorganisms derived from a living body" below, and other microorganisms such as fungi (e.g., yeasts, molds, mushrooms, and the like), mosses, microalgae, protozoans, and the like.

The "microorganisms derived from a living body" refer to all the microorganisms that normally live in every portion of a living body, including the inside of the body, the body surface, and the like of humans and other organisms (the expressions "microorganisms derived from a living body", "microorganisms isolated from a living body", "resident microorganisms", "gastrointestinal microorganisms", "resident/gastrointestinal microorganisms which reside in a living body such as human", and the like may also be used), and are classified, for example, into bacteria, fungi, viruses, and the like.

These microorganisms encompass not only microorganisms that are directly collected from nature such as a living body or the like, but also microorganisms obtained by culturing and artificially proliferating those microorganisms, mutants of those microorganisms, artificially modified microorganisms obtained through a transformation technique or other techniques, and the like.

Of the above-listed "active substances", low molecular weight compounds that have certain pharmacological effects on human bodies and animals, and microorganisms are preferable. More specifically, microorganisms derived from a living body are preferable. Even more specific example of the microorganisms derived from a living body include intestinal bacteria.

Specifically, it may be exemplified what contains at least any one kind of the following three kinds of bacteria:
(I)-(i)-1: lactic acid bacteria;
(I)-(i)-2: butyric acid bacteria; and
(I)-(i)-3: saccharification bacteria.

The "Bio-Three (registered trademark)" formulation (also referred to simply as "Bio3" hereinafter) is an example of a formulation that contains all of the above-mentioned three kinds of bacteria.

### (I)-(ii) Active Substance Protective Agent

The "(I)-(ii) active substance protective agent" refers to an additive or the like to be added in a living body in order to improve the activity retainability of an active substance (e.g., a medicine, microorganisms derived from a living body, or the like), or the handleability, formability, ease of taking, or the like thereof, and encompasses substances to be used to manufacture what is called a DDS formulation or the like.

It is thought that using the assistant agent of the present invention makes it possible to more safely and reliably protect what is called a DDS formulation and deliver it to an application site.

Specific examples of (I)-(ii) are (I)-(ii)-(a) below and the like, and these can be used alone or in combination of two or more, but there is no limitation thereto. Moreover, the "(I)-(ii) active substance protective agents" of the same kind can be used alone or in combination of two or more.

### (I)-(ii)-(a) An excipient

The (I)-(ii)-(a) excipient is not particularly limited as long as an excipient that is commonly used in a pharmaceutical formulation or the like is used, but it is preferable that use thereof in a pharmaceutical formulation or the like that includes microorganisms is established.

Specific examples thereof include substances as listed below, but an excipient used in the present invention is not limited thereto.

(I)-(ii)-(a)-1: Polyvinyl alcohol
(I)-(ii)-(a)-2: Starch
(I)-(ii)-(a)-3: Lactose and/or lactose hydrate
(I)-(ii)-(a)-4: Polyvinyl pyrrolidone
(I)-(ii)-(a)-5: Organic acid and/or organic acid salt

As the organic acid (salt) of (I)-(ii)-(a)-5, a fatty acid (salt) is preferable, and a long-chain fatty acid (salt) is more preferable. Specific examples thereof include stearic acid (a stearate) and the like.

Ratio between Active Substance and Active Substance Protective Agent such as an excipient oer the like in "Complex Containing Active Substance (IV)"

In the present invention, there is no particular limitation on the ratio between the "(I)-(i) active substance" and the "(I)-(ii) active substance protective agent" as typified by the "(I)-(ii)-(a) excipient", and the ratio that is normally used in a pharmaceutical formulation can be employed.

Specifically, the total amount of "(I)-(i)" in 1 g of the total mass of "(I)-(i)" and "(I)-(ii)-(a)" is preferably, for example, 5 mg (0.5 mass%) or more, more preferably 10 mg (1 mass%) or more, even more preferably 50 mg (5 mass%) or more, and particularly preferably 100 mg (10 mass%) or more.

The "(I)-(ii)-(a) excipient" protects the "(I)-(i) active substance" through, for example, a method in which the "(I)-(ii)-(a) excipient" is mixed with the "(I)-(i) active substance" in advance to form (IV) below before the "(I)-(i) active substance" is introduced into a living body with the "assistant agent for assisting introduction into a living body" or "DDS assistant agent" of the present invention, or before the "(I)-(i) active substance" and the "assistant agent for assisting introduction into a living body" are mixed together to form the "composition containing an active substance" of the present invention.

(IV) Complex (i.e., complex, mixture, and so on) containing an active substance that includes (I)-(i) and (I)-(ii)-(a)

### Other Kinds of (I)-(ii) Active Substance Protective Agent

Examples of the "(I)-(ii) active substance protective agent" other than an excipient include, for example, (I)-(ii)-(b) to (I)-(ii)-(h) below.

(I)-(ii)-(b) metal particle
(I)-(ii)-(c) micelle forming substance
(I)-(ii)-(d) liposome forming substance
(I)-(ii)-(e) antibody for forming ADC (Antibody Drug Conjugate)
(I)-(ii)-(f) PEG (polyethylene glycol) modification agent
(I)-(ii)-(g) micellar nanoparticle (block polymer of polyethylene glycol and poorly water-soluble polyamino acid)
(I)-(ii)-(h) microcapsule forming substance

"Metal fine particles" are preferably used as the "metal particles". Metal fine particles can form metal colloidal particles in the (II) solvent and protect the "(I)-(i) active substance".

Specific examples of the kind of metal that constitutes the metal particles include, for example, platinum (Pt), gold (Au), silver (Ag), palladium (Pd), copper (Cu), nickel (Ni), indium (Id), and the like. In particular, platinum particles are preferable because they have excellent biological compatibility.

The "(I)-(ii) active substance protective agent" may be introduced into a living body with the "assistant agent for assisting introduction into a living body" or "DDS assistant agent" of the present invention in the state, where the "(I)-(ii) active substance protective agent" is formed to "complex of (IV)" above by mixing with the "(I)-(i) active substance" together or exists alone separately from the "(I)-(i) active substance" . Also, the "(I)-(ii) active substance protective agent" can be dispersed as one component of the "assistant agent for assisting introduction into a living body" or "DDS assistant agent" of the present invention in the "(II) solvent" in advance.

It should be noted that the above-described "(IV) complex containing an active substance" may also contain various additives and the like in addition to the "(I)-(i) active substance" and the "(I)-(ii) active substance protective agent" as long as the object of the present invention is not hindered.

Moreover, the "composition containing an active substance" of the present invention may also contain various additives and the like in addition to (I)-(i) or the "DDS assistant agent", (II), and (III) as long as the object of the present invention is not hindered.

Dosage Form of "(IV) Complex Containing Active Substance"

When the "(I)-(ii)-(a) excipient (vehicle)" is used as the "(I)-(ii) active substance protective agent", examples of the dosage form of the "(IV) complex containing an active substance" include "powder (powdered drug)", "granules", a "tablet", a "D tablet (disintegrating tablet)", an "OD tablet (orally disintegrating tablet)", and the like. From the viewpoint that "nano-sized or smaller gas bubbles of (III)" can easily cover the surface or enter the inside of (I) through gaps or the like in the surface and protect the active substance from the inside, the dosage form is preferably "powder", "granules", or a "tablet", "D tablet", "OD tablet" or the like that is not coated with sugar coating or the like, and more preferably "powder".

It should be noted that the following are specific examples of the "(IV) complex containing an active substance".

"Bio3" (Bio-Three (registered trademark))-containing powder (powdered drug (powder))

### Active substance (intestinal bacteria):

Saccharification bacteria 50 mg + lactomin (lactic acid bacteria) 10 mg + butyric acid bacteria 50 mg / 1 g (one packet)

### Active substance protective agent (b) (excipient):

(I)-(ii)-1: Polyvinyl alcohol (completely saponified)
(I)-(ii)-2: Potato starch
(I)-(ii)-3: Lactose hydrate
(I)-(ii)-4: Povidone (polyvinyl pyrrolidone)

It should be noted that a "Bio3-containing tablet" and a "Bio3-containing OD tablet (orally disintegrating tablet)", which contain the same active substance as that of the "powdered drug" above but are different therefrom in the content of the active substance, the excipients, and the dosage form, are also available. These can also be used as the "complex containing an active substance" in the present invention.

### "Bio3"-containing tablet

### Active substance (intestinal bacteria):

Saccharification bacteria 10 mg + lactomin (lactic acid bacteria) 2 mg + butyric acid bacteria 10 mg / 200 mg (one tablet)

### Active substance protective agent (b) (excipient):

(I)-(ii)-1: Polyvinyl alcohol (completely saponified)
(I)-(ii)-2: Potato starch
(I)-(ii)-3: Lactose hydrate
(I)-(ii)-4: Povidone (polyvinyl pyrrolidone)
(I)-(ii)-5: Magnesium stearate

### "Bio3"-containing OD tablet

### Active substance (intestinal bacteria):

Saccharification bacteria 10 mg + lactomin (lactic acid bacteria) 2 mg + butyric acid bacteria 10 mg / 100 mg (one tablet)

### Active substance protective agent (b) (excipient):

(I)-(ii)-2: Potato starch
(I)-(ii)-3: Lactose hydrate
(I)-(ii)-6: Talc
(I)-(ii)-7: Sodium stearyl fumarate
(I)-(ii)-8: Anhydrous calcium hydrogen phosphate
(I)-(ii)-9: Low-substituted hydroxypropyl cellulose
(I)-(ii)-10: Crospovidone
(I)-(ii)-11: Light anhydrous silicic acid

### Method for Manufacturing (IV) Complex Containing Active Substance

The "(IV) complex containing an active substance" can be manufactured using, for example, a method described in the description of a "step (1)" of a "method for manufacturing a composition containing an active substance" of the present invention, which will be described later,or the like.

### Method for Manufacturing Composition Containing Active Substance

The "composition containing an active substance" of the present invention can be manufactured using the "method for manufacturing a composition containing an active substance" of the present invention, which will be described later.

### Application of Composition Containing Active Substance

The above-mentioned "composition containing an active substance" of the present invention can be used as a "composition for preventing and/or treating a disease or improving a physical constitution and/or physical condition" of the present invention, which will be described later.

### Composition for Preventing and/or Treating Disease or Improving Physical Constitution and/or Physical Condition of the Present Invention

The "composition for preventing and/or treating a disease or improving a physical constitution and/or physical condition" of the present invention is characterized by including the above-described "composition containing an active substance" of the present invention.

The "composition for preventing and/or treating a disease or improving a physical constitution and/or physical condition" of the present invention can be used to prevent and/or treat various diseases and improve verious physical conditions or physical constitutions that are not very good but are not considered as being sick.

The diseases are not particularly limited to specific diseases, and examples thereof include diseases that can be prevented or treated with an "active substance that can be formulated using a certain active substance protective agent". Examples of diseases for which such an active substance is effective include gastrointestinal dysfunction such as gastroenteritis, diabetes, and atopic dermatitis.

### Method for Manufacturing Composition Containing Active Substance of the Present Invention

The "method for manufacturing a composition containing an active substance" of the present invention can be manufactured using a manufacturing method that includes at least the following steps (1) to (3).

(1) a step of preparing (I)-(i) protected by (I)-(ii)
(2) a step of generating gas bubbles of (III) in a solvent of (II)
(3) a step of dispersing and/or dissolving (I)-(i) in (II)

(I)-(i) an active substance
(I)-(ii) an active substance protective agent
(II) a solvent
(III) nano-sized or smaller (smaller than 1 micron) gas bubbles

### Description of Step (1)

"Preparing (I)-(i) protected by (I)-(ii)" encompasses purchasing a known drug or the like that is formulated using "(I)-(ii)" such as a "(I)-(ii)-(a) excipient" from the market, manufacturing "(I)-(i)" protected by "(I)-(ii)" using "(I)-(i)" and "(I)-(ii)" in accordance with a known drug manufacturing method etc., and the like. For example, "(I)-(i) protected by (I)-(ii) can be manufactured as the "(IV) complex containing an active substance" or the like by mixing the "(I)-(i) active substance", the "(I)-(ii)-(a) excipient", and other components, and then, for example, drying and molding the mixture as needed to formulate the mixture into various powder, granules, tablets, or the like.

Moreover, (1) can also be performed simultaneously in the step (2) or (3).

That is, it is sufficient that, before the "composition containing an active substance" is finally completed, by, for example, mixing "(I)-(i)" and "(I)-(ii)" as appropriate in the process of (2) or (3), "(I)-(i)" is protected by "(I)-(ii) and (III).

### Order of Steps

It should be noted that the steps above need not be necessarily performed in the order of (1), (2), and (3). In particular, (1) can be performed separately from (2) and (3), or in parallel with (2) and (3).

(2) and (3) need not be performed in this order, but it is preferable to perform (2) and (3) in this order from the viewpoint that a risk of deactivation of the active substance during the manufacturing process can be minimized.

(1) may be performed independently of (2) and (3), but a configuration is also possible in which (I)-(ii) is dispersed or dissolved in (II) in advance and is caused to exhibit the protective effect in the step (3).

In such a case, the following methods can be employed as (2), for example: a method in which (I)-(ii) is dispersed or dissolved in (II) and then gas bubbles of (III) are generated; a method in which gas bubbles of (III) are generated in (II) and then (I)-(ii) is dispersed or dissolved; a method in which a step of dispersing or dissolving (I)-(ii) in (II) and a step of generating gas bubbles of (III) in (II) are performed independently of each other, and then a mixing step is performed; or the like.

### Method for Preventing and/or Treating Disease or Improving Physical Constitution and/or Physical Condition of the Present Invention

A "method for preventing and/or treating a disease or improving a physical constitution and/or physical condition" of the present invention can be performed by introducing the above-described "composition containing an active substance" of the present invention into a living body.

As described in the description of the "assistant agent for assisting introduction into a living body" of the present invention, "introduction into a living body" means that the introduced active substance is brought into a state of being capable of exhibiting its function, efficacy, or effect in a living body by physically binding the composition of the present invention to the living body through engraftment, attachment, adsorption, or the like as well as, for example, by allowing the composition of the present invention to be present inside or outside the blood vessels, in cavities in the living body (in the intestinal tract), and the like, for example. Substantially all known introduction techniques including direct injection techniques for blood vessels, subcutaneous tissues, and intracutaneous tissues can be employed.

### Method for Causing Active Substance to Exhibit Function, Efficacy, or Effect in Living Body of the Present Invention

A "method for causing an active substance to exhibit a function, efficacy, or effect thereof in a living body" of the present invention can be performed by introducing "(I)-(i)" below together with "(I)-(ii)" below in the living body by using the "assistant agent for assisting introduction into a living body" of the present invention:
(I)-(i) an active substance; and
(I)-(ii) an active substance protective agent.

The following methods are specific examples of the "method of performing introduction by using an assistant agent for assisting introduction into a living body", for example: a method in which "(I)-(i)" is dispersed or dissolved in the "assistant agent for assisting introduction into a living body" together with "(I)-(ii)" just before introduction into a living body and then the resulting mixture is introduced into a living body; a method in which the "assistant agent for assisting introduction into a living body" is administered to a living body independently of "(I)-(i)" and "(I)-(ii)" simultaneously with or at a time point slightly different from the administration of "(I)-(i)" and "(I)-(ii)"; or the like.

The reason why the "assistant agent for assisting introduction into a living body" may be administered independently of "(I)-(i)" and "(I)-(ii)" is as described in the description of "Application of Assistant Agent for Assisting Introduction into Living Body" above.

As described in the description of the "assistant agent for assisting introduction into a living body" of the present invention, "introduction into a living body" means that the introduced active substance is brought into a state of being capable of exhibiting its function, efficacy, or effect in a living body by physically binding the composition of the present invention to the living body through engraftment, attachment, adsorption, or the like as well as, for example, by allowing the composition of the present invention to be present inside or outside the blood vessels, in cavities in the living body (in the intestinal tract), and the like, for example. Substantially all known introduction techniques including direct injection techniques for blood vessels, subcutaneous tissues, and intracutaneous tissues can be employed.

### Examples

Hereinafter, the present invention will be more specifically described by way of examples, comparative examples, and the like, but the present invention is not limited to these examples.

It should be noted that a "method for detecting microorganisms" used in the examples and the like will be described prior to describing the examples and the like.

### Method for Detecting Microorganisms

The number of microorganisms and the kinds thereof were determined using a known "ribosome profiling (16S ribosome RNA (rRNA) sequence)" and a known "next-generation sequencer (NGS)" in combination.

It should be noted that, in addition to the "ribosome profiling", "shotgun metagenomics sequence", which is a technique for comprehensively analyzing all the genes of all microorganisms present in a subject, or the like can also be used as a profiling technique, but the "ribosome profiling", which can be performed with a small number of sequencing trials and at low cost, was used.

### Ribosome Profiling

The "ribosome profiling" is a technique that is widely used for identification, classification, and the like of microbial species and the like in recent years and in which identification of gene transcription products that are being translated is utilized. By sequencing the "16S rRNA gene" present in most microorganisms, the microbial species present in a subject can be identified and the abundance ratios thereof can be estimated, and therefore, this technique is suitable for analysis of bacterial flora (microbiota: microbial population), which has been conventionally considered to be difficult to analyze, and is particularly suitable for analysis of intestinal bacterial flora, which is considered to include about 40 trillion intestinal bacteria.

### Next-Generation Sequencer (NGS)

The "next-generation sequencers" were developed in the US around 2000. With these apparatuses, a significantly large number of base sequences of DNA fragments can be simultaneously read out compared with conventional DNA sequencers. Illumina in the US etc. took the lead in the development thereof.

Examples of the next-generation sequencers manufactured by Illumina include, for example, DNA sequencers of "MiSeq series", "NextSeq series" ,"HiSeq series" manufactured by Illumina, and the like. The principles of these sequencers, the method of using these sequencers, and the like are disclosed in the website having the following URL.

https://jp.illumina.com/landing/s/metagenome.html

Of the sequencers above, the "MiSeq series" sequencer was used in the present invention.

### Example A: Assistant Agent A for Assisting Introduction into Living Body (Containing Nano-Sized or Smaller Gas Bubbles)

### Materials and Methods

### Manufacturing of "Nanobubble Water A"

An "assistant agent A for assisting introduction into a living body (nanobubble water A)" of the present invention (Example A) was produced using mineral water and air as materials with the above-described apparatus and the like.

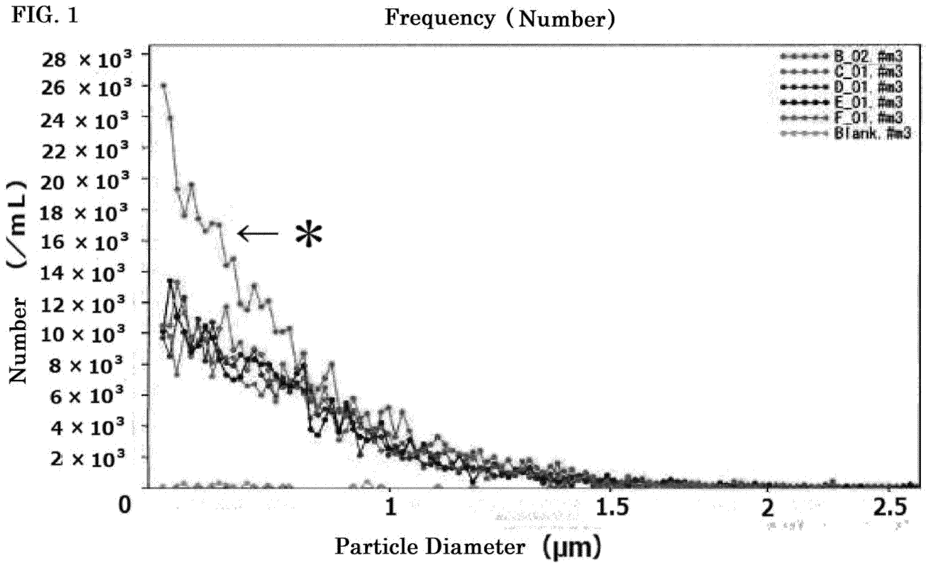

The "nanobubble water A" produced as mentioned above was diluted 250 fold for the convenience of measurement of the diameters of the gas bubbles, and the size of the gas bubbles, the number of the gas bubbles, and the like in the diluted solution were measured using "Multisizer 3" manufactured by Beckman Coulter. FIG. 1 shows the results.

The uppermost line graph indicated by "*" in the diagram shows the results from the analysis of the "nanobubble water A".

Analysis Results Shown in FIG. 1: Average diameter of gas bubbles: 830 nm; Number of gas bubbles: about 435 thousand per milliliter

Accordingly, it is considered that the number of the gas bubbles in 1 ml of the "nanobubble water A" (before diluted 250 fold) used in the present invention was approximately a hundred million.

### Example B: Assistant Agent B for Assisting Introduction into Living Body (Containing Nano-Sized or Smaller Gas Bubbles)

### Materials and Methods

An "assistant agent B for assisting introduction into a living body (nanobubble water B)" of the present invention (Example B) was manufactured in the same manner as in Example A, except that hydrogen and air were used together as gas to be enclosed when gas bubbles were generated, and hydrogen was enclosed in the latter stage of the enclosing process to increase the hydrogen concentration in gas bubbles.

It should be noted that the amount of hydrogen used in the enclosing process was about 250 times as large as the amount of air.

It is considered that the number of the gas bubbles in 1 ml of the "nanobubble water B" (not diluted 250 fold) used in the present invention was also approximately a hundred million.

### Comparative Example C: Assistant Agent C for Assisting Introduction into Living Body (Containing No Nano-Sized or Smaller Gas Bubbles)

A physiological saline solution in which no nano-sized or smaller gas bubbles were enclosed was used as an assistant agent C for assisting introduction into a living body of Comparative Example C.

### Example 1: Composition Containing Active Substance (Excipient Type)

A "composition containing an active substance" of Example 1 of the present invention was obtained by dissolving or dispersing 1 g of a "complex containing an active substance" below in 100 ml of the above-mentioned "assistant agent B for assisting introduction into a living body" of Example B.

### Complex Containing Active Substance

"Bio3" (Bio-Three (registered trademark))-containing powder (powdered drug / manufactured and sold by TOA Biopharma Co., Ltd.)

### Active Substance (Intestinal Bacteria):

Saccharification bacteria 50 mg + lactomin (lactic acid bacteria) 10 mg + butyric acid bacteria 50 mg / 1 g (one packet)

### Active Substance Protective Agent (b) (Excipient):

(I)-(ii)-1: Polyvinyl alcohol (completely saponified)
(I)-(ii)-2: Potato starch
(I)-(ii)-3: Lactose hydrate
(I)-(ii)-4: Povidone (polyvinyl pyrrolidone)

### Comparative Example 1: Composition Containing Active Substance

A "composition containing an active substance" of Comparative Example 1 was obtained by dissolving or dispersing 1 g of a "complex containing an active substance" that was the same as that used in Example 1 in 100 ml of the above-mentioned "assistant agent for assisting introduction into a living body" of Comparative Example C.

### Test Example 1: Test to Confirm Effects of Assistant Agent for Assisting Introduction into Living Body (1) / Intestinal Bacteria Introduction (Transplantation) Test on Mouse

The "composition containing an active substance" of Example 1 or Comparative Example 1 was administered to a mouse in accordance with the following procedure.

### Materials and Methods

### Administration Target

Four 5-week-old male ICR mice (versatile albino mice for medical purposes) were prepared and were divided into two groups, namely an example administration group (two mice) and a comparative example administration group (two mice).

The mice of the example administration group were respectively named Ex-1-1 Mouse and Ex-1-2 Mouse.

The mice of the comparative example administration group were respectively named Com-1-1 Mouse and Com-1-2 Mouse.

### Administration Method

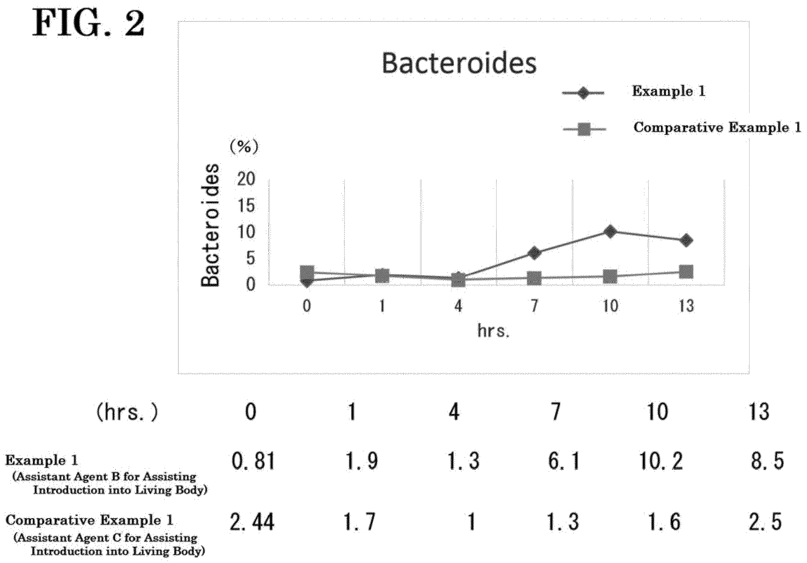

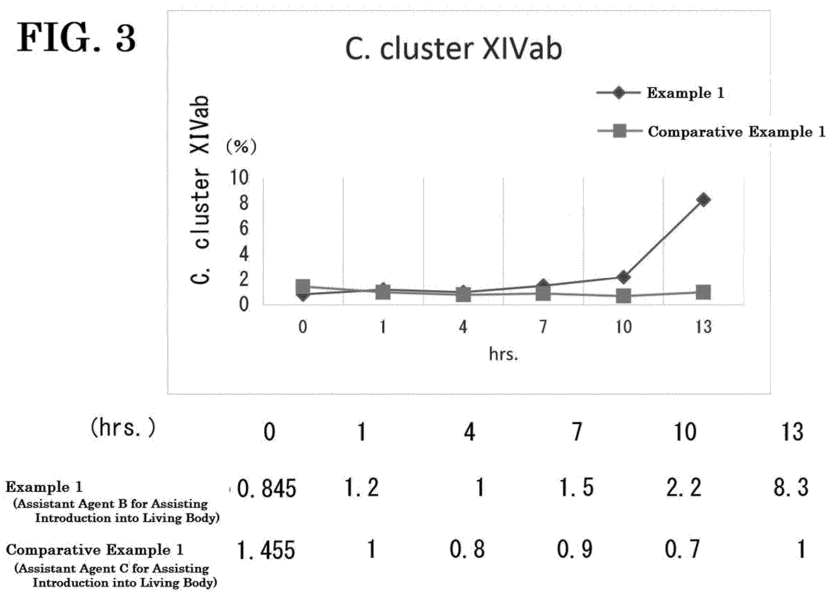

1 ml of the "composition containing an active substance" of Example 1 or Comparative Example 1 was administered to each mouse via the anus using an intestinal infusion technique, and the mouse was observed for 13 hours. FIGS. 2 and 3 shows the results.

For the bacterial flora analysis, feces that were first collected after the elapse of a predetermined time (0 hours (i.e., before administration), 1 hour, 4 hours, 7 hours, 10 hours, and 13 hours) were used to analyze bacterial flora therein. The analysis was performed in accordance with the above-described description in "Method for Detecting Microorganisms".

It should be noted that % in FIGS. 2 and 3 refers to the ratio (based on the number) of intestinal bacteria of the kind indicated in the figures to the entire bacterial flora, and the values in the figures are average values of the respective groups.

### Results

As shown in FIG. 2, the ratio of "Bacteroides" to the entire bacterial flora in the feces (intestinal flora) of the example administration group (the average of Ex-1-1 Mouse and Ex-1-2 Mouse) obviously increased compared with that of the comparative example administration group (the average of Com-1-1 Mouse and Com-1-2 Mouse).

Also, as shown in FIG. 3, the ratio of "Clostridium cluster XIVab" to the entire bacterial flora in the feces (intestinal flora) of the example administration group (the average of Ex-1-1 Mouse and Ex-1-2 Mouse) obviously increased compared with that of the comparative example administration group (the average of Com-1-1 Mouse and Com-1-2 Mouse).

### Discussion

"Bacteroides" are also called opportunistic bacteria. It is known that "Bacteroides" increase due to a metabolic change caused by administration of a drug, and the like.

Accordingly, it is thought that the results shown in FIG. 2 mean that the active substance "intestinal bacteria" in Example 1 and "secretions or metabolic products" thereof were directly or indirectly (i.e., via the excipient, the other components, and the like) protected by the nano-sized or smaller gas bubbles contained in Example 1 and could thus be reliably delivered to the vicinity of the intestinal mucosa, and therefore, the existing indigenous lactic acid bacteria dramatically decreased from 41.2% to 3.1% (not shown) and "the genus Bacteroides" appeared instead, that is, the intestinal bacteria were replaced (the intestinal flora balance changed).

"Clostridium cluster XIVab" is a group that includes "butyric acid (producing) bacteria", which were used as one kind of active substance in Example 1 administered.

Accordingly, it is thought that the results shown in FIG. 3, namely "'Clostridium cluster XIVab' significantly increased" , mean that one kind of active substance "butyric acid bacteria" in Example 1 and "secretions or metabolic products" thereof were directly or indirectly (i.e., via the excipient, the other components, and the like) protected by the nano-sized or smaller gas bubbles contained in Example 1 and could thus be reliably delivered to the vicinity of the intestinal mucosa, and as a result, not only "butyric acid bacteria" but also the entire "Clostridium cluster XIVab" to which "butyric acid bacteria" belong increased steadily in a living body. The reason for this is that, when a certain microorganism increases, related microorganisms having similar properties are likely to increase simultaneously.

That is, the results shown in FIGS. 2 and 3 indicate that the "complex containing an active substance" that contained the "active substance protective agent (excipient)" and the "active substance (microorganisms)" could be more reliably introduced into a living body (the "composition containing an active substance" of the present invention was introduced into a living body) by using the "assistant agent for assisting introduction into a living body" of the present invention, and that the "assistant agent for assisting introduction into a living body" of the present invention can function as a "DDS assistant agent".

It should be noted that, in view of the fact that the "complex containing an active substance" used in the test example above was "intestinal bacteria in which the active substance was covered or protected by the excipient", it is thought that, when formulated (protected) by using the "active substance protective agent" such as an excipient or the like, not only microorganisms but also all substances that have activity useful for a living body, such as various organic compounds ranging from low molecular weight molecules to high molecular weight molecules, and inorganic substances (e.g., minerals) , or the like, can be more efficiently introduced into a living body by using the "assistant agent for assisting introduction into a living body" or "DDS assistant agent" of the present invention, compared with the case where a simple solvent (e.g., physiological saline solution) that contains no "nano-sized or smaller gas bubbles" is used.

In particular, even in the case of small active substances (e.g., low molecular weight organic compounds or the like) that are considered to be difficult to cover by "nano-sized or smaller gas bubbles" without being processed, increasing the size thereof to a certain extent (a size capable of being covered by a plurality of nano-sized or smaller gas bubbles) by using the active substance protective agent such as an excipient or the like enables efficient introduction into a living body with the "assistant agent for assisting introduction into a living body" of the present invention.

Although not necessarily applicable to every case since the specific size depends on the diameter of gas bubbles in the "assistant agent for assisting introduction into a living body", the inner diameter of at least a portion in the entire "active substance" protected by the "active substance protective agent" is, for example, preferably several nm or more, and more preferably several ten nm or more. This is preferable because such an active substance is considered to be capable of being smoothly covered by gas bubbles having a nano-sized or smaller average diameter (smaller than 1 µm).

### Example 2: Composition Containing Active Substance (Metal Fine particle (Metal Nanocolloid) Type)

A "composition containing an active substance" of the present invention is manufactured by dissolving an active substance of 1) at a concentration of 10 ng/ml in an "assistant agent for assisting introduction into a living body" of the present invention in which metal colloids are generated by dispersing metal fine particles of 4) at a concentration of one hundred thousand fine particles/ml in 100 ml of a solvent of 2) below in which 3) below has been generated.

1) active substance: insulin
2) solvent: mineral water
3) nano-sized or smaller gas bubbles: average diameter of 100 to 200 nm, a hundred million gas bubbles/ml
4) metal fine particles: platinum

### Functions

With the above-mentioned "assistant agent for assisting introduction into a living body" and "composition containing an active substance" of the present invention, it is thought that the "active substance" is protected by the "nano-sized or smaller gas bubbles" and the "platinum nanocolloids" and can thus be reliably delivered to the vicinity of the intestinal mucosa when administered via the anus or the like or can pass through the stomach while maintaining the activity when orally administered, compared with the case where the active substance is protected by only the "nano-sized or smaller gas bubbles".

The reason for this is that it is thought that a better function of protecting the active substance from gastric acid or the like can be exhibited due to protection by both colloids and nano-sized gas bubbles.

### Test Example 2: Test to Confirm Effects of Assistant Agent for Assisting Introduction into Living Body (2) / Measurement of Bacterial Component Recognition Receptor mRNA Expression Level in Intestinal Epithelial Cells

Intestinal epithelial cells have various "receptors" that each recognize "at least one of bacterial components (all components derived from bacteria, such as constituent components of cell walls of bacterial cells, intracellular proteins, DNA in the nuclei), and the like " derived from intestinal bacteria, and it is thought that the "amount of intestinal bacteria that interact (have cross-talk) with intestinal epithelial cells" correlates with the "bacterial component recognition receptor expression levels" to some extent.

That is, it is thought that the "effect of introducing intestinal bacteria (active substance) into a living body (the level of the effect of the assistant agent for assisting introduction into a living body)" can be estimated by measuring the "bacterial component recognition receptor mRNA levels" in intestinal epithelial cells before and after the addition of the "intestinal bacteria (active substance)" to intestinal epithelial cells (stimulation of the intestinal epithelial cells by the "intestinal bacteria (active substance)").

Accordingly, after a complex containing an active substance" had been added to cultured intestinal epithelial cells together with an "assistant agent for assisting introduction into a living body", the mRNA expression levels of "bacterial component recognition receptors", "major response molecules" of the receptors, and the like were measured.

### Materials and Methods

### Complex Containing Active Substance

"Bio3 formulation (powdered drug)" (also referred to simply as "Bio3" hereinafter)

### Assistant Agent for Assisting Introduction into Living Body

### Example B: "assistant agent B for assisting introduction into a living body" (nanobubble water B)

### Intestinal Epithelial Cells / CaCO-2:

CaCO-2 cells produced as follows were used: human colon carcinoma RCB0988 cells (cells derived from human colon carcinoma) imported from ECACC (European Collection of Authenticated Cell Cultures) via an agency (K.A.C.) were introduced into the designated special culture medium below, and were then cultured in an incubator (5% CO₂, 37°C).

### Designated Special Culture Medium:

MEM (Minimum Essential Media) + 20% FBS (Fetal Bovine Serum) + 0.1 mM NEAA (Non-Essential Amino Acids)

It should be noted that ultrapure water was used as a liquid component.

### Method of Preculture:

CaCO-2 cells were precultured at a concentration of fifty thousand cells/ml in 2 ml of the culture medium (one hundred thousand cells in total) in a 35-mm petri dish for 48 hours, and were then used in subsequent experiments.

Method of Adding "Complex Containing Active Substance" and "Assistant Agent for Assisting Introduction into Living Body"

The old culture solution in the petri dish used in the preculture was removed, and the cell surfaces were washed with PBS (Phosphate Buffered Saline). Then, 2 ml each of a control culture medium or example culture medium below was added, and culture was continued.

### Control Culture Medium:

A culture solution (containing no "Bio3") that was produced by replacing the liquid component of the designated special culture medium with the "assistant agent B for assisting introduction into a living body (nanobubble water B)" of Example B

### Example Culture Medium:

A culture solution that was produced by further adding "Bio3" powder (complex containing an active substance) to the control culture medium above such that the concentration of the Bio3 was 0.1 wt%

It should be noted that the following description is the reason why the concentration of the "Bio3" powder was set to 0.1 wt% in the description above.

As a "preparatory experiment", the mRNA expression levels were measured 4 hours after the addition of the culture medium at concentrations of the "Bio3" powder between 1 wt% and 0.0001 wt% inclusive adjusted through ten-fold serial dilution.

As a result, the "mRNA expression level when the 'assistant agent B for assisting introduction into a living body (nanobubble water B)' of Example B was used" tended to vary depending on the concentration of "Bio3", and it was determined that such a tendency could be estimated by adding 0.1 wt% of "Bio3".

### Extraction of Total RNA

Total RNA was extracted from cells in each petri dish by using Trizol (registered trademark) Reagent (ThermoFisher Scientific) in accordance with the known product protocol 1, 4, or 8 hours after the addition of the above-mentioned culture medium.

Before the experiment below was performed, the RNA concentration in a portion of each specimen (1 pl) was quantified by using NanoDrop (ThermoFisher Scientific) in order to set the total RNA concentrations to be the same.

### Measurement of mRNA Expression Level

### Measurement Method:

Quantitative RT-PCR (RT-qPCR)

### Template:

500 ng of total RNA obtained as described above

### Reagents for One Step RT-PCR:

Luna Universal One-Step RT-qPCR kit

It should be noted that "One step RT-PCR" is a system in which cDNA synthesis by reverse transcription reaction (Reverse Transcriptase; RT reaction) and quantitative PCR are performed in one test tube.

### PCR Apparatus:

Thermal Cycler Dice Real Time System II (manufactured by Takara)

### Measurement Target:

mRNA of "bacterial component recognition receptors", "major response molecules" of the receptors, and the like below

It should be noted that known primers were used.

### Bacterial Component Recognition Receptors

TLR2 and TLR4

### Bacterial Component Recognition Receptors (Cytoplasmic Receptors)

Nod-1 and Nod-2

Major Response Factor Produced in Proportion to Expression of Bacterial Component Recognition Receptors (Cytoplasmic Receptors) NLRP3 and NLRC4

IL-1β

### Endogenous Control

GAPDH (housekeeping gene)

It should be noted that the concentrations of the reagents and the primers, the temperatures, the reaction cycles, and other measurement conditions were determined in accordance with the known method in which the above-described "Luna Universal One-Step RT-qPCR kit" is used.

### Results

When the control culture medium (culture solution produced using only the "nanobubble water B" without adding "Bio3") was used, mRNA showed substantially no increase in all the measurement targets.

On the other hand, when the example culture medium (culture solution to which "Bio3" and the "nanobubble water B" had been added) was used, it was observed that the mRNA levels of Nod-1, Nod-2, and IL-1β tended to increase, which peaked 4 hours after the addition of the culture medium (not shown).

### Discussion

It is thought from the results above that the increase in mRNA in this test example was not caused by nanobubble water itself but by interaction between the active substance (intestinal bacteria) and the intestinal epithelial cells increased by nanobubble water.

That is, it was suggested that the "assistant agent for assisting introduction into a living body" of the present invention is useful for introducing the "complex containing an active substance" into a living body.

Test Example 3: Test to Confirm Effects of Assistant Agent for Assisting Introduction into Living Body (3) / Confirmation of Effects of Suppressing Glucose Absorption (Uptake) by Intestinal Epithelial Cells (in vitro)

### Materials and Methods

### Complex Containing Active Substance

The above-described "Bio3" formulation (powdered drug) was used as the "complex containing an active substance (intestinal bacteria)".

### Composition Containing Active Substance

### "Composition containing an active substance" of Example 3:

An "example culture medium" (a composition containing an active substance of Example 3) was prepared by replacing the liquid component of the above-described designated special culture medium with the "assistant agent for assisting introduction into a living body" of Example B (nanobubble water B), setting the Glucose (-) (Glucose Free), and setting the content of "Bio3" formulation (powdered drug) to 0.1 wt%. The thus-obtained composition is referred to as "Bio3 with NB-(B)" hereinafter.

### "Composition Containing Active Substance" of Comparative Example 2:

A "comparative example culture medium" (a composition containing an active substance of Comparative Example 2) was prepared in the same manner as in Example 3 above, except that the liquid component of the above-described designated special culture medium was replaced with the "assistant agent for assisting introduction into a living body" of Comparative Example C (physiological saline solution). The thus-obtained composition is referred to as "Bio3 with Saline" hereinafter.

### Target of Addition of Composition Containing Active Substance and Glucose

CaCO-2 cells (intestinal epithelial cells) prepared in the same manner as those used in Test 2 were used.

### Method of Adding Composition Containing Active Substance

The method described in Mojika L et al. (Mojica L et al, Toxicology Reports 2018, vol.5, P.552-560) was modified and used as the experimental method.

Specifically, the CaCO-2 cells (5×10⁴ cells/well) seeded in a 96-well plate was cultured in "Bio3 with NB-(B)" or "Bio3 with Saline" above for 4 hours.

### Method of Adding Glucose

100 pM (µmol/L) of fluorescence-labeled glucose below, which is a glucose analog, was added to the cultured CaCO-2 cells above.

### 2-NBDG:

2-(N-(7-nitrobenz-2-oxa-1,3-diazol-4-yl)amino)-2-deoxy-D-glucose

(ThermoFisher, Carlsbad, California)

### Method of Measuring Glucose Absorption (Uptake) Amount

The cultured cells collected after the elapse of a predetermined time (0, 30, 60, 120, 180 minutes) since the addition of 2-NBDG were washed with ice-cold PBS to stop glucose absorption, and then the fluorescence levels (excitation wavelength Ex: 485 nm, fluorescence wavelength Em: 535 nm) of the label on 2-NBDG that had been taken up by the cells were measured using a fluorescence plate reader (SYNERGY HT micro plate reader: Bio Tek). The results are shown as relative fluorescence intensity.

It should be noted that the relative fluorescence intensity is the average value of the results from five points at each measurement time.

### Results

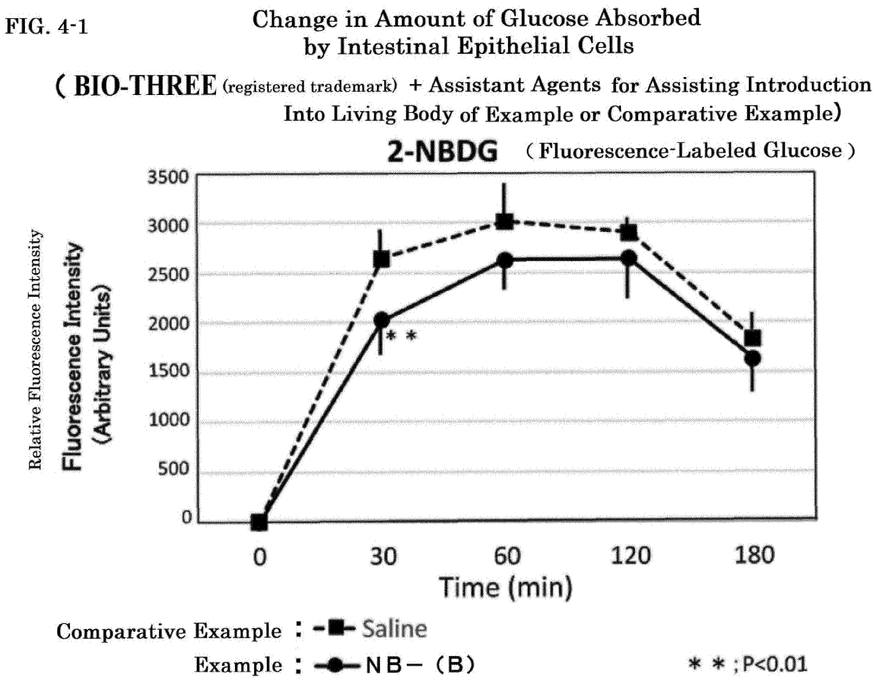

FIG. 4-1 shows the results.

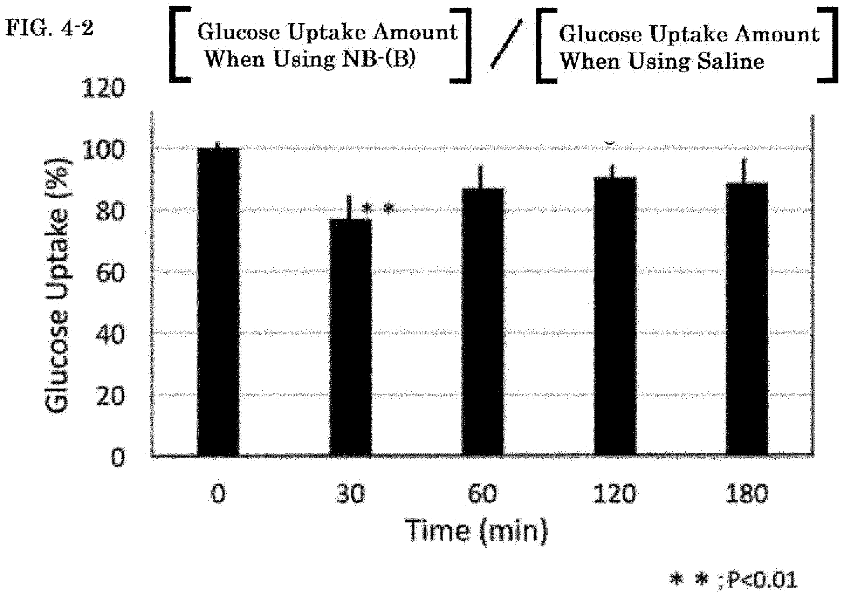

In addition, FIG. 4-2 shows the ratio of the "relative fluorescence intensity when using Example B (nanobubble water B)" to the "relative fluorescence intensity when using Comparative Example C (physiological saline solution)" at each time point shown in FIG. 4-1.

It was revealed from FIGS. 4-1 and 4-2 that the amount of glucose uptake into intestinal tract (small intestine) epithelial cells was further suppressed or the rate of glucose uptake thereinto was further slowed in the case where "Bio3" was introduced into the cultured cells by using the "nanobubble water" of the present invention compared with the case where a physiological saline solution was used (30 minutes after the addition of glucose: P<0.01 (significantly different)).

### Discussion

It is thought that the reason why the result "the amount of glucose uptake into intestinal epithelial cells was suppressed or the rate of glucose uptake thereinto was slowed" above was obtained is that cross-talk between intestinal bacteria and intestinal tract (small intestine) epithelial cells was more likely to occur in the case where the "intestinal bacteria protected by an excipient (Bio3)" were added with the "assistant agent B for assisting introduction into a living body" of Example B (nanobubble water B) compared with the case where the "assistant agent C for assisting introduction into a living body" of Comparative Example C (physiological saline solution) was used.

The reason for this is that it is suggested that butyric acid secreted by butyric acid bacteria, which is one of the three kinds of intestinal bacteria contained in "Bio3" used in the test example above, is particularly likely to suppress an increase in the blood glucose level, and that it is also reported that butyric acid bacteria promote secretion of intestinal hormones (e.g., GLP-1) and thus the functions of insulin is enhanced.

That is, it is thought from the test result "the amount of glucose uptake into intestinal epithelial cells was suppressed and the rate of glucose uptake thereinto was slowed due to the addition of Bio3 with nanobubble water B" that the "assistant agent for assisting introduction into a living body" (nanobubble water) of the present invention more effectively assists introduction of an active substance protected by an excipient, such as "Bio3", into a living body than a "physiological saline solution" does, and can enhance the effects expected for "Bio3", namely the effects of reducing the blood glucose level and thus preventing and treating diabetes.

### Test Example 4: Test to Confirm Effects of Assistant Agent for Assisting Introduction into Living Body (4) / Test for Measurement of Blood Glucose Level and Plasma Insulin Level in Diabetic Disease Model Mouse (in vivo)

The "complex containing an active substance" was injected into the intestine of a STZ-induced diabetes model mouse (STZ-mouse) together with the "assistant agent for assisting introduction into a living body", and then measurement of a "change in plasma glucose (blood glucose level) (Test Example 4-1)" and "measurement of the plasma insulin level (Test Example 4-2)" at the end of the treatment were performed.

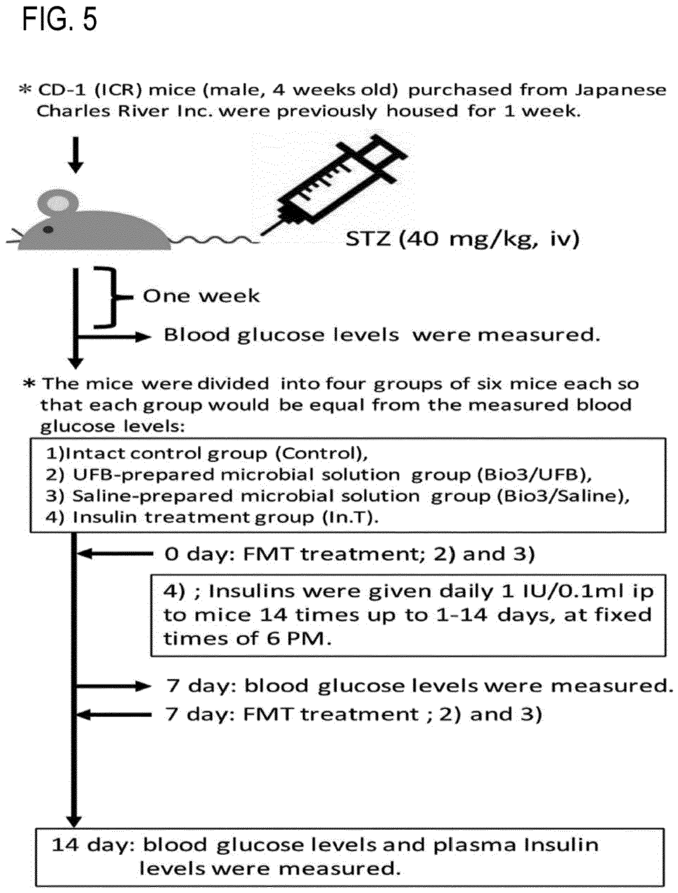

The measurement tests were performed in accordance with the schematic diagram shown in FIG. 5.

In addition, the experiments were performed in accordance with the Animal Experiment Guidelines of Research Organization of Biological Activity.

### Materials and Methods

### Complex Containing Active Substance

"Bio3" formulation (powdered drug)

### Assistant Agent for Assisting Introduction into Living Body

Comparative Example C: "assistant agent C for assisting introduction into a living body" (Physiological Saline Solution)

Example B: "assistant agent B for assisting introduction into a living body" (nanobubble water B)

### Composition Containing Active Substance

"Compositions containing an active substance" below were prepared by mixing the above-described "active substance complex (Bio3 formulation)" with the respective "assistant agents for assisting introduction into a living body" above, and were then used in the experiments.

Composition containing an active substance of Example 4: "Bio3 with NB-(B)"

Composition containing an active substance of Comparative Example 3: "Bio3 with Saline"

Insulin as listed below was purchased and used as a positive control instead of the "composition containing an active substance".

### Mouse insulin (FUJIFILM Wako Pure Chemical Corporation)

### STZ-mice

Diabetes model mice "STZ-mice" were produced as follows.

### Mice

CD-1 (ICR) mice (male, 4 weeks old) purchased from Charles River Japan were preliminarily kept for 1 week before experiment.

### STZ

Streptozotocin (STZ): FUJIFILM Wako Pure Chemical Corporation

### Procedure for Producing Disease Model Mice

STZ (40 mg/kg) prepared in a citrate buffer (100 mmol/L, pH 4.5) was intravenously administered (iv) to the tail veins of the above-mentioned mice.

### Grouping of Disease Model Mice

One week after, blood was collected from ophthalmic venous plexus of the mice with a capillary, and the mice were divided into the following four groups (six mice per group) based on the measured blood glucose levels such that the average blood glucose levels of the groups were equal to one another as follows.

1) Non-treated control group (Control):
   Average blood glucose level 452±31.2 (mg/dl)
2) "Bio3 with NB-(B)" administration group:
   Average blood glucose level 451±39.2 (mg/dl)
3) "Bio3 with Saline" administration group:
   Average blood glucose level 451±39.2 (mg/dl)
4) Insulin administration group (Insulin treatment: positive control):
   Average blood glucose level 456.3±57.1 (mg/dl)

It should be noted that other molecular biology reagents were purchased from FUJIFILM Wako Pure Chemical Corporation and used.

Procedure for Administrating "Composition Containing Active Substance (Bio3)" etc.

1 ml of "Bio3 with NB-(B)" or "Bio3 with Saline" per mouse was administered to the large intestine via the anus with a single-administrate needle on the starting day of the experiment and on Day 7, that is, twice.

It should be noted that, in FIG. 5, "NB-(B)" is expressed as "UFB (ultrafine bubble)", "Bio3 with NB-(B)" is expressed as "Bio3/UFB", "Bio3 with Saline" is expressed as "Bio3/Saline", and the "Bio3" administration with a needle is expressed as "FMT".

1 IU/0.1 ml of "insulin" serving as the positive control was intraperitoneally administered (ip) to the mouse routinely at 6^{:}00 PM every day from Day 1 to Day 14, that is, 14 times.

### Measurement Conditions

The blood glucose level (Test Example 4-1) and the plasma insulin level (Test Example 6-2) were respectively measured under the following conditions.

### Test Example 4-1: Measurement of Blood Glucose Level

Measurement timing: twice, on Day 7 and Day 14 after the treatment

Blood collection method: blood was collected from ophthalmic venous plexus of the mice with a capillary.

### Measurement Apparatus:

Glucose CII-test Wako (FUJIFILM Wako Pure Chemical Corporation)

### Fluorescence-Labeled Glucose Analog (Fluorescence Tracer):

"2-NBDG"

(2-(N-(7-Nitrobenz-2-oxa-1,3-diazol-4-yl)Amino)-2-Deoxyglucose, ThermoFisher, Carlsbad, California)

### Test Example 4-2: Measurement of Plasma Insulin

Blood Collection Method: On Day 14, blood was collected from the jugular vein under ether anesthesia, and the measurement was performed.

The plasma insulin level was measured using Ultra Sensitive Mouse Insulin ELISA Kit (Morinaga Institute of Biological Science, Inc.).

### Results

### Test Example 4-1

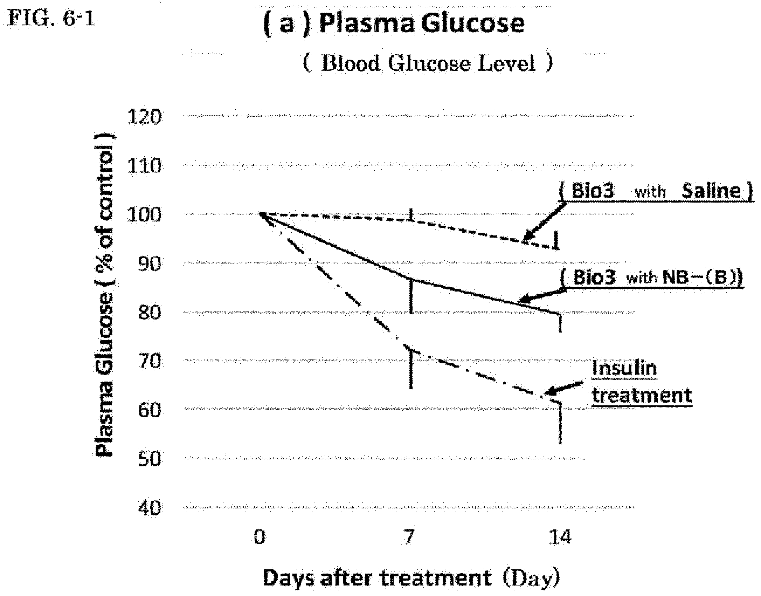

The results were shown as the ratios to the non-treated control group. The blood glucose level of the "Bio3 with NB-(B)" administration group significantly decreased (FIG. 6-1).

On the other hand, the "Bio3 with Saline" administration group was not different from the non-treated control group (FIG. 6-1).

Specifically, on Day 14 after the treatment, the blood glucose level of the insulin administration group serving as the positive control decreased to up to 60%, whereas the blood glucose level of the "Bio3 with NB-(B)" administration group decreased to about 80%.

### Test Example 4-2

The plasma insulin levels of the respective groups at the end of the experiment were as low as the detection limit, but were slightly different.

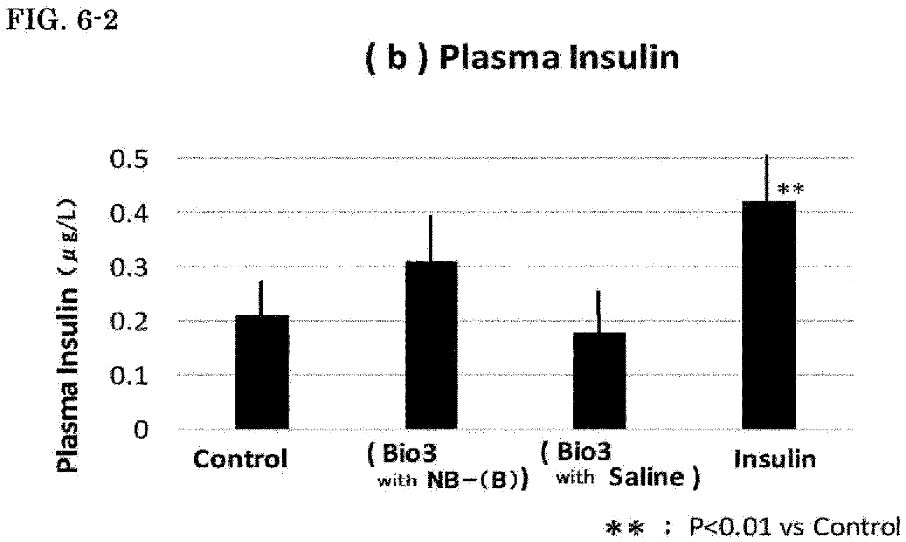

Specifically, the plasma insulin level of the "Bio3 with NB-(B)" administration group was not as high as that of the "insulin" administration group serving as the positive control, but showed the tendency of being obviously higher than that of the non-treated control group (FIG. 6-2).

On the other hand, the plasma insulin level of the "Bio3 with Saline" administration group was substantially the same as that of the non-treated control group, and was not so much different therefrom (FIG. 6-2).

That is, it was revealed that the "complex containing an active substance (Bio3)" introduced into the mouse by using the "assistant agent for assisting introduction into a living body" of the present invention increased the plasma insulin, though such an effect was slightly milder than that of the positive control in which "insulin" was directly administered through injection.

### Discussion

In view of the results of Test Examples 4-1 and 4-2, it is thought that, when "Bio3" was protected by fine gas bubbles in the "nanobubble water", the "Bio3" recognition ability of intestinal epithelial cells were improved, resulting in the increase in the plasma insulin level and the decrease in the blood glucose level.

That is, these results are considered to suggest that the "assistant agent for assisting introduction into a living body" of the present invention may widen the applicability of oral drugs, enteric drugs, and the like, and may pave the way for a treatment method simpler than the common diabetes treatment method (insulin injection).

### Industrial Applicability

The assistant agent for assisting introduction into a living body of the present invention can be used as an assistant agent for a drug delivery system capable of more reliably delivering a complex containing an active substance that includes an excipient (what is called a pharmaceutical formulation etc.) to the inside of a living body.

The composition containing an active substance of the present invention can be more reliably introduced to the inside of a living body.

The composition for preventing and/or treating a disease or improving a physical constitution and/or physical condition of the present invention can more reliably exhibit a function, efficacy, or effect in a living body, and is thus useful in preventing and/or treating a disease or improving a physical constitution and/or physical condition.

## Claims

1. An assistant agent for assisting introduction into a living body, the assistant agent to be used to introduce (I)-(i) together with (I)-(ii) into a living body, and comprising (II) and (III) below:
(I)-(i) an active substance;
(I)-(ii) an active substance protective agent;
(II) a solvent; and
(III) nano-sized or smaller (smaller than 1 micron) gas bubbles.

2. The assistant agent for assisting introduction into a living body according to claim 1, wherein a gas component in the gas bubbles comprises one or two or more kinds of gasses listed below:
(i) air;
(ii) hydrogen;
(iii) nitrogen;
(iv) ozone;
(v) oxygen;
(vi) carbon dioxide; and
(vii) argon.

3. The assistant agent for assisting introduction into a living body according to claim 1 or 2, wherein (I)-(ii) active substance protective agent is at least one kind of protective agent listed below:
(I)-(ii)-(a) an excipient.

4. The assistant agent for assisting introduction into a living body according to any one of claims 1 to 3, wherein (I)-(i) active substance includes at least one or more kinds of microorganisms.

5. The assistant agent for assisting introduction into a living body according to claim 4, wherein at least one kind of the microorganism is a microorganism derived from a living body.

6. The assistant agent for assisting introduction into a living body according to claim 5, wherein at least one kind of the microorganism derived from a living body is an intestinal bacterium.

7. The assistant agent for assisting introduction into a living body according to any one of claims 1 to 3, wherein (I)-(i) active substance includes at least one or more kinds of organic compounds.

8. The assistant agent for assisting introduction into a living body according to any one of claims 1 to 7, further comprising (I)-(ii) below:
(I)-(ii) an active substance protective agent.

9. An assistant agent for a drug delivery system, comprising (II) and (III) below:
(II) a solvent; and
(III) nano-sized or smaller (smaller than 1 micron) gas bubbles.

10. A composition containing an active substance, comprising (I)-(i) to (III) below:
(I)-(i) an active substance;
(I)-(ii) an active substance protective agent;
(II) a solvent; and
(III) nano-sized or smaller (smaller than 1 micron) gas bubbles.

11. A composition for preventing and/or treating a disease or improving a physical constitution and/or physical condition, comprising the composition according to claim 10.

12. A method for manufacturing the composition according to claim 10 or 11, comprising at least steps of (1) to (3) below:
(1) a step of preparing (I)-(i) protected by (I)-(ii);
(2) a step of generating gas bubbles of (III) in a solvent of (II); and
(3) a step of dispersing and/or dissolving (I)-(i) in (II),
where
(I)-(i) is an active substance;
(I)-(ii) is an active substance protective agent;
(II) is a solvent; and
(III) is nano-sized or smaller (smaller than 1 micron) gas bubbles.

13. A method for preventing and/or treating a disease or improving a physical constitution and/or physical condition, comprising a step below:
a step of introducing the composition according to claim 11 into a living body.

14. A method for causing an active substance to exhibit a function, efficacy, or effect thereof in a living body, comprising a step below:
a step of introducing (I)-(i) below together with (i)-(ii) below into a living body by using the assistant agent for assisting introduction into a living body according to any one of claims 1 to 9
(I)-(i) an active substance; and
(I)-(ii) an active substance protective agent.
